# FASCICULE DE BREVET EUROPEEN

(11) **EP 1 372 766 B1**
(45) Date de publication et mention de la délivrance du brevet: **24.02.2010**
(21) Numéro de dépôt: 02722375.9
(22) Date de dépôt: 26.03.2002
(51) Int. Cl.: A61M 5/28, A61M 5/315

(54) **SERINGUE POUR SOLUTIONS VISCOELASTIQUES**
SPRITZE FÜR VISKOELASTISCHE LÖSUNGEN
SYRINGE FOR VISCOELASTIC SOLUTIONS

(30) Priorité: 27.03.2001 FR 0104090
(43) Date de publication de la demande: 02.01.2004
(73) Titulaire: Laboratoire de Contactologie Appliquée - LCA, 28000 Chartres (FR)
(72) Inventeur: VINCENT, Patrice, F-28130 Mevoisins (FR)
(74) Mandataire: CAPRI
(86) Numéro de dépôt international: PCT/FR2002/001035
(87) Numéro de publication internationale: WO 2002/076534

(56) Documents cités:
- EP-A- 0 695 555
- EP-A- 0 974 320
- WO-A-02/11793
- FR-A5- 2 110 516
- US-A- 4 702 737
- US-A- 4 929 230
- US-A- 5 865 799
- US-A1- 2002 035 351

## Description

La présente invention concerne une seringue pour solutions viscoélastiques utilisées dans les domaines médicaux et chirurgicaux. De telles solutions sont actuellement utilisées en rhumatologie sous forme d'injections intra-articulaires, en dermatologie pour le comblement des rides, en chirurgie plastique, en chirurgie abdominale, et en chirurgie ophtalmologique, sans que cette liste d'applications soit forcément limitée dans le temps.

Une seringue selon le préambule de la revendication 1 est connue du document EP-A-0 974 320.

Plus particulièrement, la présente invention peut s'appliquer lors de la chirurgie de la cataracte, avec pose d'une lentille intraoculaire. Dans ce cas, le rôle des solutions viscoélastiques est de créer les espaces nécessaires aux gestes chirurgicaux, et de protéger les tissus oculaires, en particulier l'endothélium cornéen. Une condition importante est que ce type de solution doit être retiré par aspiration à la fin de l'intervention, sous peine de risquer de provoquer une augmentation néfaste de la pression intraoculaire.

Dans le cadre de la chirurgie de la cataracte, on utilise généralement d'abord un produit présentant une plus forte viscosité dynamique à taux de cisaillement élevé. Ceci est notamment le cas au cours des étapes chirurgicales appelées capsulorhexis et phacoémulsification, qui sont des techniques chirurgicales bien connues de l'homme du métier. Un produit présentant une viscosité dynamique plus faible pour des conditions de cisaillement similaires, est de préférence utilisé en second, lors de la pose de la lentille intraoculaire.

Actuellement, le chirurgien doit manipuler deux seringues contenant chacune une solution viscoélastique distincte. L'autre possibilité est de n'utiliser qu'une seule solution viscoélastique. Dans le premier cas, l'opération est plus compliquée de par l'utilisation de plusieurs seringues, et dans le second cas, la solution utilisée, à défaut de constituer un bon compromis, présente au moins un inconvénient, à savoir soit une mauvaise tenue en place durant la phacoémulsification, soit des difficultés d'extraction en fin d'intervention, après la pose de la lentille intraoculaire.

La présente invention a pour but de fournir une seringue pour solutions viscoélastiques dans le domaine médical qui ne reproduit pas les inconvénients susmentionnés.

En particulier, la présente invention a pour but de fournir une seringue pour solutions viscoélastiques qui simplifie la tâche du chirurgien, et qui permet d'utiliser des produits présentant les caractéristiques optimales à chaque étape de l'intervention.

La présente invention a également pour but de fournir une telle seringue pour solutions viscoélastiques qui soit simple et peu coûteuse à fabriquer et à assembler.

La présente invention a donc pour objet un dispositif pour distribuer des solutions viscoélastiques tel que décrit dans la revendication 1.

Des modes de réalisation avantageux sont décrits dans les revendications dépendantes.

La présente invention a également pour objet l'utilisation d'une seringue telle que décrite ci-dessus dans la chirurgie de la cataracte.

D'autres caractéristiques et avantages de la présente invention apparaîtront plus clairement au cours de la description détaillée suivante de deux modes de réalisation avantageux de celle-ci, faite en référence aux dessins joints, donnés à titre d'exemples non limitatifs, et sur lesquels,
- la figure 1 est une vue schématique en section transversale d'une seringue, et
- la figure 2 est une vue schématique en section transversale d'une seringue non couverte par la présente invention.

De manière générale, une seringue 1 comporte un corps de seringue 2 généralement cylindrique comportant d'une part un orifice de distribution 3 et d'autre part un piston 4 actionné par une tige d'actionnement 5. L'utilisateur appuie sur la tige d'actionnement 5 pour déplacer le piston 4 à l'intérieur du corps 2 pour distribuer le produit contenu dans la seringue à travers l'orifice de distribution 3.

Selon l'invention, la seringue 1 comporte deux solutions viscoélastiques V1, V2 qui présentes des propriétés différentes, lesdites solutions V1, V2 étant distribuées successivement, dans un ordre prédéterminé.

Plus particulièrement, les deux solutions viscoélastiques V1, V2 se distinguent par leur nature chimique, et/ou le poids moléculaire de leur principe actif et/ou la concentration de ce principe actif. Plus généralement, les solutions viscoélastiques de la présente invention présentent des propriétés rhéologiques différentes, et avantageusement elles présentent des viscosités dynamiques différentes, pour des taux de cisaillement élevés.

Selon l'invention, on prévoit d'interposer un diaphragme mobile 10 entre les solutions V1, V2. Ce diaphragme 10 comporte de préférence des joints d'étanchéité 15 sur sa périphérie externe qui coopèrent avec le corps le corps 2 de la seringue 1. Ainsi, ce diaphragme 10 peut coulisser contre la paroi interne du corps de la seringue de manière étanche, tout en étant guidé comme le piston de la seringue. Au fur et à mesure que la première solution viscoélastique V1 est distribuée, le diaphragme 10 se déplace en même temps que le piston 4, sous l'effet de la poussée qui est transmise par la seconde solution viscoélastique V2. En variante, on peut imaginer une liaison mécanique temporaire reliant le diaphragme 10 au piston 4 pour déplacer simultanément ces deux éléments pendant l'expulsion de la première solution viscoélastique V1, cette liaison mécanique étant supprimée au moment de la distribution de la seconde solution viscoélastique V2.

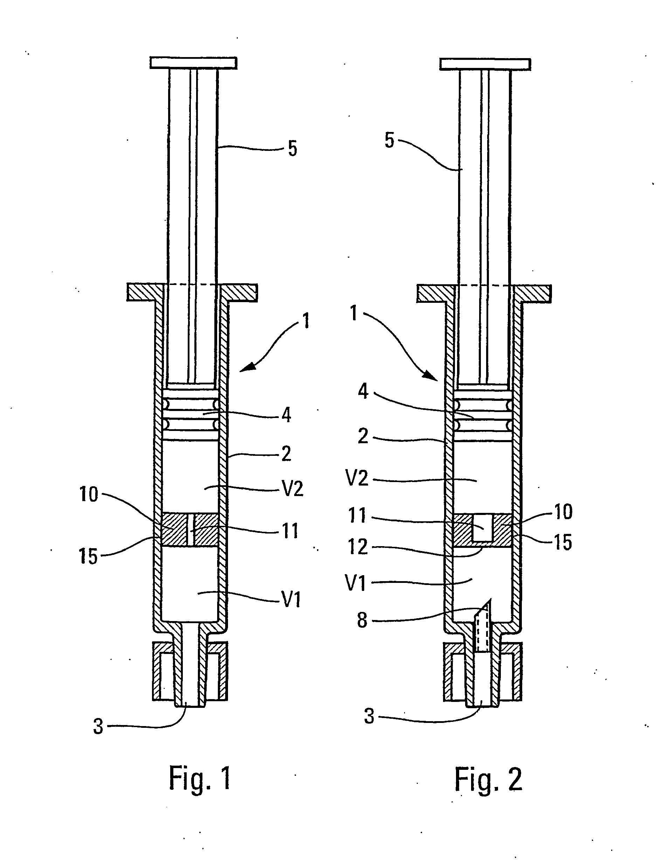

Pour permettre la distribution de la seconde solution viscoélastique V2, le diaphragme 10 comporte une ouverture 11, de préférence disposée de manière centrale, de préférence de faible dimension. Ceci est généralement suffisant pour que les solutions V1 et V2 ne se mélangent avant que la solution V1 soit entièrement distribuée, comme représenté dans l'exemple de la figure 1, même si cette ouverture 11 n'est pas obturée.

Ainsi, lorsque l'utilisateur appuie sur la tige d'actionnement 5, la première solution V1 est d'abord expulsée à travers l'orifice de distribution 3, puis le diaphragme arrive en butée dans le fond du corps 2 de la seringue et alors la seconde solution viscoélastique V2 est expulsée à travers l'ouverture 11 du diaphragme 10. Pour un bon fonctionnement, la force à exercer pour provoquer le passage de la seconde solution viscoélastique V2 à travers la petite ouverture 11 doit généralement être supérieure au frottement du diaphragme 10 contre la paroi de la seringue, en tenant compte de l'éventuelle présence de la liaison mécanique. La présence d'une petite interface d'air à l'intérieur de l'ouverture 11, par exemple une micro bulle d'air captive, permet avantageusement d'éviter tout contact entre les deux solutions jusqu'à leur utilisation.

Dans le mode non couvert par la présente invention représenté sur la figure 2, le diaphragme 10 est représenté de manière tout à fait étanche, avec une membrane 12 qui obture l'ouverture 11, qui dans ce cas peut être de dimension plus grande que dans le mode de réalisation représenté sur la figure 2. Cette mise en oeuvre peut être envisage dans le cas où les solutions V1 et V2 auraient tendance à se mélanger, par exemple par simple rééquilibrage osmotique ou au cours de la distribution de la première solution V1. La seringue 1 comporte alors des moyens d'ouverture, tel qu'une pointe de percement 8, qui sont adaptés à ouvrir ladite membrane 12 lorsque le diaphragme 10 arrive en contact avec le fond de la seringue, c'est à dire lorsque la première solution viscoélastique V1 qui est disposée en aval du diaphragme a été totalement distribuée.

De cette manière, le contact est totalement empêché entre les deux solutions V1 et V2 avant et pendant l'actionnement du dispositif.

Comme représenté schématiquement sur les dessins, la seringue peut comporter au niveau de son ouverture de distribution 3 un système de sécurité Luer-Lock qui permet de verrouiller une canule ou une aiguille d'injection sur l'embout de la seringue.

Comme mentionné précédemment, une utilisation particulièrement avantageuse de la présente invention concerne la chirurgie de la cataracte, mais la présente invention n'est pas limitée à cette utilisation.

Bien que la présente invention ait été décrite en référence à deux modes de réalisation avantageux de celle-ci, elle n'est pas limitée par les exemples représentée, et l'homme du métier peut y apporter toutes modifications sans sortir du cadre de la présente invention, telle que définie par les revendications annexées.

## Revendications

1. Dispositif pour distribuer des solutions viscoélastiques consistant en une seringue (1) formée d'un unique corps qui contient deux solutions viscoélastiques (V1, V2) présentant des propriétés différentes, lesdites solutions (V1, V2) étant distribuées successivement, dans un ordre prédéterminé, le dispositif étant **caractérisé en ce que** lesdites deux solutions viscoélastiques (V1, V2) sont physiquement séparées par un unique diaphragme mobile interposé entre lesdites deux solutions viscoélastiques (V1, V2), ledit diaphragme mobile (10) incorporant une ouverture centrale (11), de préférence de petite dimension, permettant l'expulsion de la solution viscoélastique (V2) disposée en amont du diaphragme (10) après distribution totale de la solution viscoélastique (V1) disposée en aval du diaphragme (10), ladite ouverture (11) étant non-obturée.

2. Dispositif selon la revendication 1, dans lequel ledit diaphragme (10) comporte des joints d'étanchéité (15).

3. Dispositif selon la revendication 1 ou 2, dans lequel une micro bulle d'air est disposée dans ladite ouverture (11) du diaphragme (10), pour éviter tout contact entre les solutions viscoélastiques (V1, V2).

4. Dispositif selon l'une quelconque des revendications précédentes, dans lequel ledit diaphragme (10) est relié par une liaison mécanique temporaire au piston (4) de la seringue pendant la distribution de la solution viscoélastique (V1) disposée en aval du diaphragme (10), ladite liaison mécanique temporaire étant supprimée après distribution totale de ladite solution viscoélastique (V1) pour permettre la distribution de la solution viscoélastique (V2) disposée en amont du diaphragme (10).

5. Dispositif selon l'une quelconque des revendication précédentes, dans lequel lesdites au moins deux solutions viscoélastiques (V1, V2) présentent des propriétés rhéologiques différentes.

6. Dispositif selon l'une quelconque des revendication précédentes, dans lequel lesdites deux solutions viscoélastiques (V1, V2) présentent des compositions chimiques et/ou des poids moléculaires de leur principe actif et/ou des concentrations de leur principe actif différents.

7. Dispositif selon l'une quelconque des revendications précédentes, dans lequel lesdites deux solutions viscoélastiques présentent des viscosités dynamiques différentes, à taux de cisaillement élevés.

## Claims

1. A device for dispensing viscoelastic solutions, said device consisting of a syringe (1) formed by a single body which contains two viscoelastic solutions (V1, V2) having different properties, said solutions (V1, V2) being dispensed in succession, in a predetermined order, the device being **characterized in that** said two viscoelastic solutions (V1, V2) are physically separated by a single moving diaphragm (10), provided between said two viscoelastic solutions (V1, V2), said moving diaphragm (10) incorporating a preferably small central opening (11) enabling the viscoelastic solution (V2) disposed upstream from the diaphragm (10) to be discharged after the viscoelastic solution (V1) disposed downstream from the diaphragm (10) has been dispensed in full, said opening (11) being not obturated.

2. A device according to claim 1, in which said diaphragm (10) includes sealing gaskets (15).

3. A device according to claim 1 or claim 2, in which a small air bubble is disposed in said opening (11) in the diaphragm (10) so as to prevent any contact between the viscoelastic solutions (V1, V2).

4. A device according to any preceding claim, in which said diaphragm (10) is connected via a temporary mechanical coupling to the plunger (4) of the syringe during dispensing of the viscoelastic solution (V1) disposed downstream from the diaphragm (10), said temporary mechanical coupling being eliminated once said viscoelastic solution (V1) has been dispensed in full, so as to enable the viscoelastic solution (V2) disposed upstream from the diaphragm (10) to be dispensed.

5. A device according to any preceding claim, in which said at least two viscoelastic solutions (V1, V2) have different rheological properties.

6. A device according to any preceding claim, in which said two viscoelastic solutions (V1, V2) have different chemical compositions and/or different molecular weights of their active principles and/or different concentrations of their active principles.

7. A device according to any preceding claim, in which said two viscoelastic solutions have dynamic viscosities that are different at high shear rates.

## Patentansprüche

1. Vorrichtung zur Ausgabe von viskoelastischen Lösungen, bestehend aus einer Spritze (1), die aus einem einzelnen Körper gebildet ist, der zwei viskoelastische Lösungen (V1, V2) enthält, die verschiedene Eigenschaften aufweisen, wobei die Lösungen (V1, V2) nacheinander in einer vorbestimmten Reihenfolge ausgegeben werden, wobei die Vorrichtung **dadurch gekennzeichnet ist, dass** die beiden viskoelastischen Lösungen (V1, V2) physikalisch durch eine einzelne bewegliche Membran getrennt sind, die zwischen den beiden viskoelastischen Lösungen (V1, V2) angeordnet ist, wobei die bewegliche Membran (10) eine mittige Öffnung (11), vorzugsweise von kleiner Größe, aufweist, die das Austreiben der viskoelastischen Lösung (V2), die oberhalb der Membran (10) angeordnet ist, ermöglicht, nachdem die viskoelastische Lösung (V1), die unterhalb der Membran (10) angeordnet ist, vollständig ausgegeben wurde, wobei die Öffnung (11) nicht verschlossen ist.

2. Vorrichtung nach Anspruch 1, wobei die Membran (10) Dichtungen (15) aufweist.

3. Vorrichtung nach Anspruch 1 oder 2, wobei eine Mikroluftblase in der Öffnung (11) der Membran (10) angeordnet ist, um jeglichen Kontakt zwischen den viskoelastischen Lösungen (V1, V2) zu unterbinden.

4. Vorrichtung nach einem der vorhergehenden Ansprüche, wobei die Membran (10) durch eine vorübergehende mechanische Verbindung während der Ausgabe der viskoelastischen Lösung (V1), die oberhalb der Membran (10) angeordnet ist, mit dem Kolben (4) der Spritze verbunden ist, wobei die vorübergehende mechanische Verbindung nach Ausgabe der gesamten viskoelastischen Lösung (V1) gelöst wird, um die Ausgabe der viskoelastischen Lösung (V2), die unterhalb der Membran (10) angeordnet ist, zu ermöglichen.

5. Vorrichtung nach einem der vorhergehenden Ansprüche, wobei die mindestens zwei viskoelastischen Lösungen (V1, V2) verschiedene rheologische Eigenschaften aufweisen.

6. Vorrichtung nach einem der vorhergehenden Ansprüche, wobei die beiden viskoelastischen Lösungen (V1, V2) verschiedene chemische Zusammensetzungen und/oder Molekulargewichte ihres Wirkstoffes und/oder Konzentrationen ihres Wirkstoffes aufweisen.

7. Vorrichtung nach einem der vorhergehenden Ansprüche, wobei die beiden viskoelastischen Lösungen verschiedene dynamische Viskositäten (Scherviskositäten) mit erhöhten Scherraten aufweisen.
